# EUROPÄISCHE PATENTANMELDUNG

(11) **EP 2 263 659 A1**
(43) Veröffentlichungstag der Anmeldung: **22.12.2010**
(21) Anmeldenummer: 10008750.1
(22) Anmeldetag: 27.05.2005
(51) Int. Cl.: A61K 9/36, A61K 31/519, A61K 31/565

(54) **Folsäurehaltiges Kontrazeptivum**

(30) Priorität: 28.05.2004 DE 102004026671; 28.05.2004 DE 102004026670
(62) Teilanmeldung aus: 05750712.1
(71) Anmelder: Grünenthal GmbH, 52078 Aachen (DE)
(72) Erfinder: Schramm, Georg, Dr., 52224 Stolberg (DE); Pâques, Eric-Paul, Prof. Dr., 52076 Aachen (DE)
(74) Vertreter: Kutzenberger, Helga

(57) **Zusammenfassung**

Die vorliegende Erfindung betrifft eine Darreichungsform zur hormonalen Kontrazeption enthaltend eine bestimmte Anzahl von hormonhaltigen Tages-Einheiten und eine bestimmte Anzahl von hormonfreien Tages-Einheiten zur ununterbrochenen, täglichen, oralen Verabreichung, wobei die hormonhaltigen Tages-Einheiten jeweils höchstens 200 µg an Folsäure und die hormonfreien Tages-Einheiten > 200 µg bis zu der bei Frauen maximal zulässigen Menge an Folsäure enthalten.

## Beschreibung

Die vorliegende Erfindung betrifft eine Darreichungsform zur hormonalen Kontrazeption enthaltend eine bestimmte Anzahl von hormonhaltigen Tages-Einheiten und eine bestimmte Anzahl von hormonfreien Tages-Einheiten zur ununterbrochenen, täglichen, oralen Verabreichung an Frauen, **dadurch gekennzeichnet, dass** die hormonhaltigen Tages-Einheiten Folsäure in einer Tagesmenge bis höchstens 200 µg und die hormonfreien Tages-Einheiten jeweils Folsäure in einer Tagesmenge > 200 µg bis zu der bei Frauen maximal zulässigen Menge an Folsäure enthalten.

Es wird vermutet, dass eine lang andauernde Einnahme von hormonalen Kontrazeptiva auf Basis von Gestagenen zu einem Mangel an Folsäure führen kann. Dieser Mangel kann beispielsweise zu cardiovaskulären Erkrankungen führen.

Außerdem ist bekannt, dass, sofern es kurze Zeit nach Abbruch der Einnahme solcher hormonaler Kontrazeptiva zu einer Schwangerschaft kommt, die Gefahr besteht, dass der Mangel an Folsäure bei dem Embryo zu Neuralrohrdefekten führen kann. Da das Neuralrohr in den ersten Wochen der Schwangerschaft entwickelt wird, ist es besonders vorteilhaft, eine präkonzeptionelle Einnahme von Folsäure zu gewährleisten.

Wenn daher wegen eines Kinderwunsches die Einnahme von hormonalen Kontrazeptiva unterbrochen wird und es bereits im ersten Zyklus nach dem Absetzen der hormonalen Kontrazeptiva zu einer Schwangerschaft kommt, ist die Gewährleistung einer entsprechend hohen Menge an Folsäure für die Zeit direkt nach dem Absetzen der sogenannten Pille besonders wichtig.

Es besteht daher ein Bedarf, hormonale Kontrazeptiva mit Folsäure in einer solchen Art und Weise auszurüsten, dass der Folsäure-Zusatz den unterschiedlichen Bedürfnissen über die Zeit während eines Einnahmezyklus und danach angepasst ist.

Aus WO 99/53910 ist bereits die Kombination von hormonalen Kontrazeptiva und Folsäure bekannt. Die Folsäuremenge pro hormonaler Tagesdosis wird nur entsprechend dem unterschiedlichen Bedarf an Folsäure gemäß fortschreitendem Alter geändert. Es wird aber nicht der unterschiedliche Bedarf an Folsäure über den Einnahmezyklus eines Kontrazeptivums berücksichtigt.

Daher war es Aufgabe der vorliegenden Erfindung, eine Darreichungsform zur hormonalen Kontrazeption zur Verfügung zu stellen, die den unterschiedlichen Bedarf an Folsäure während des hormonalen Einnahmezyklus und einer darauffolgenden Einnahme hormonfreier Tages-Einheiten berücksichtigt.

Diese Aufgabe wurde durch die Bereitstellung der erfindungsgemäßen Darreichungsform zur hormonalen Kontrazeption enthaltend eine bestimmte Anzahl von hormonhaltigen Tages-Einheiten und eine bestimmte Anzahl von hormonfreien Tages-Einheiten zur ununterbrochenen, täglichen, oralen Verabreichung an Frauen, **dadurch gekennzeichnet, dass** die hormonhaltigen Tages-Einheiten Folsäure in einer Tagesmenge bis höchstens 200 µg und die hormonfreien Tages-Einheiten jeweils Folsäure in einer Tagesmenge > 200 µg bis zu der bei Frauen maximal zulässigen Menge an Folsäure enthalten, gelöst.

Frauen im gebärfähigen Alter haben einen täglichen Bedarf an Folsäure, der durch eine gesunde Ernährung ausreichend gedeckt werden kann. Eine lang andauernde Einnahme von hormonalen Kontrazeptiva enthaltend Gestagene kann zu einem zusätzlichen Bedarf an Folsäure führen, der ebenfalls durch eine gesunde Ernährung gedeckt werden kann. Allerdings ist dafür eine tägüche Gabe der bei Frauen minimal wirksamen Tagesmenge an Folsäure empfehlenswert.

Dementsprechend können die hormonhaltigen Tages-Einheiten der erfindungsgemäßen Darreichungsform jeweils eine Tagesmenge an Folsäure aufweisen, die diese minimal wirksame Tagesmenge an Folsäure mit umfasst. Vorzugsweise enthalten die hormonhaltigen Tages-Einheiten der erfindungsgemäßen Darreichungsform 0 bis 200 µg Folsäure, besonders bevorzugt 5 bis 200 µg Folsäure.

Die hormonhaltigen Tages-Einheiten der erfindungsgemäßen Darreichungsform können auch keinen Zusatz an Folsäure aufweisen, wobei aber ein Zusatz von Folsäure bevorzugt ist.

Um bei einem angestrebten Kinderwunsch möglichst rasch die notwendige Menge an Folsäure bzw. den erhöhten Bedarf an Folsäure zumindest bei Beginn einer Schwangerschaft zu gewährleisten, und dabei einer mögliche Schädigung des Embryos durch Folsäuremangel vorzubeugen, enthalten die hormonfreien Tages-Einheiten der erfindungsgemäßen Darreichungsform Folsäure in einer Menge von mehr als 200 µg bis zur maximal zulässigen Tagesmenge an Folsäure für Frauen, vorzugsweise bis zu 5 mg Folsäure pro Tages-Einheit, besonders bevorzugt von mehr als 200 µg bis 5 mg Folsäure, ganz besonders bevorzugt bis zu der bei fertilen Frauen maximal zulässige Tagesmenge an Folsäure.

Durch den Zusatz von Folsäure zu den hormonfreien Tageseinheiten der erfindungsgemäßen Darreichungsform in Mengen bis zur maximal für fertile Frauen zulässigen Menge kann schon während der Einnahme der hormonfreien Tages-Einheiten die Folsäurekonzentration im weiblichen Körper soweit erhöht sein, dass gegebenenfalls bei einem Absetzen der Einnahme eines hormonhaltigen Kontrazeptivums und einer darauffolgenden Schwangerschaft möglichst frühzeitig der zu Beginn einer Schwangerschaft erhöhte Folsäurebedarf im weiblichen Körper gewährleistet ist.

Vorzugsweise enthalten die hormonhaltigen Tages-Einheiten der erfindungsgemäßen Darreichungsform jeweils dieselbe Menge Folsäure. Dies gilt auch für die hormonfreien Tages-Einheiten, die ebenfalls jeweils dieselbe, gegenüber den hormonhaltigen Tages-Einheiten aber erhöhte Menge Folsäure aufweisen.

Die Folsäure kann in der erfindungsgemäßen Darreichungsform auch als pharmazeutisch unbedenkliches Salz, vorzugsweise als Natrium-, Kalium- oder Magnesium-Salz oder als ein entsprechendes Derivat vorliegen.

Als Derivate der Folsäure eignen sich Mono- oder Diester, wobei bei Diestern eine unterschiedliche oder identische Veresterung vorliegen kann. Vorzugsweise eignet sich als Alkoholwert eine niedrige. Alkylgruppe mit C₁-C₈, wie Methyl, Ethyl, Propyl oder Butyl, eine verzweigte, niedrige Alkylgruppe mit C₃-C₈, wie Isopropyl, Isobutyl oder sec. Butyl, eine Cycloalkyl-Gruppe, wie Cyclopentyl oder Cyclohexyl, eine ArylGruppe, wie Phenyl oder substituiertes Phenyl mit 1-2 Substituenten, z. B. wie eine niedrige Alkyl- oder Halogen-Alkoxyl-Gruppe, oder eine Arylalkyl-Gruppe mit einem C₁-C₈ Alkylrest und eine Arylgruppe, wie Phenyl oder substituiertes Phenyl.

Darüber hinaus können die hormonfreien Tages-Einheiten und gegebenenfalls die hormonhaltigen Tages-Einheiten zusätzlich zur Folsäure weitere Vitamine oder Mineralien enthalten.

Die Anzahl der Tages-Einheiten einer erfindungsgemäßen Darreichungsform kann einem natürlichen, monatlichen, weiblichen Menstruations-Zyklus entsprechen. In diesem Fall enthält die erfindungsgemäße Darreichungsform 21 bis 25 hormonhaltige Tages-Einheiten und 7 bis 3 hormonfreie Tages-Einheiten.

Es ist aber auch möglich, dass die Gesamtzahl der hormonhaltigen Tages-Einheiten mehr als einen natürlichen, weiblichen Monatszyklus entspricht, so dass eine erfindungsgemäße Darreichungsform hormonhaltige Tages-Einheiten für eine ununterbrochene Einnahme bis zu 2 Jahren, vorzugsweise bis zu 1 Jahr, und 7 bis 3 hormonfreie Tages-Einheiten enthalten kann. Es ist aber auch möglich, dass die erfindungsgemäße Darreichungsform 42 bis 52 bzw. 77 bis 193 hormonhaltige Tages-Einheiten neben 7 bis 3 hormonfreien Tages-Einheiten aufweisen kann.

Die hormonhaltigen Tages-Einheiten der erfindungsgemäßen Darreichungsform können jeweils einen Gehalt von mindestens einer kontrazeptiv wirkenden Hormonkomponente, vorzugsweise eine Kombination von Hormonkomponenten wie einem Östrogen und einem Gestagen aufweisen.

Für die hormonhaltigen Tages-Einheiten der erfindungsgemäßen Darreichungsform eignen sich Östrogene, die vorzugsweise aus der Gruppe umfassend Oestradiol, Estradiolvalerat, Ethinylestradiol und Mestranoi ausgewählt sind. Als Östrogen für die erfindungsgemäße Darreichungsform ist Ethinylestradiol besonders bevorzugt.

Für die hormonhaltigen Tages-Einheiten der erfindungsgemäßen Darreichungsform eignen sich Gestagene, die vorzugsweise aus der Gruppe umfassend Norethisteron, Norethisteronacetat, Norethisteronenantat, Norgestimat, Norgestrel, Levonorgestrel, Gestoden, Hydroxyprogesteroncaproat, Medroxyprogesteronacetat, Megestrolacetat, Chlormadinonacetat, Lynestrenol, Cyproteronacetat, Drospirenon, Dienogest, Desogestrel, Progesteron, Dydrogesteron, Medrogeston, Ethynodiol, Promegeston, Nomegestrolacetat und Trimegeston ausgewählt sind.

Die Hormone werden vorzugsweise in den nachstehend angegebenen Mengen verwendet.

### Östrogene:

| | |
|---|---|
| Estradiol, Estradiolvalerat | 0,5 bis 4 mg |
| Ethinylestradiol | 5 bis 50 µg |
| Mestranol | 8 bis 70 µg |

### Gestagene:

| | |
|---|---|
| Norethisteron, -acetat | 0,5 bis 1,0 mg |
| Norgestimat | 0,1 bis 0,25 mg |
| Norgestrel | 0,3 bis 1,0 mg |
| Levonorgestrel | 0,05 bis 0,15 mg |
| Gestoden | 0,05 bis 0,12 mg |
| Hydroxyprogesteroncaproat | 10 bis 800 mg |
| Medroxyprogesteronacetat | 2,5 bis 40 mg |
| Megestrolacetat | 1,0 bis 10 mg |
| Chlormadinonacetat | 0,5 bis 10 mg |
| Lynestrenol | 0,4 bis 3 mg |
| Cyproteronacetat | 0,5 bis 10 mg |
| Drospirenon | 1,0 bis 10 mg |
| Dienogest | 1,0 bis 10 mg |
| Desogestrel | 0,06 bis 0,30 mg |
| Progesteron | 100 bis 1000 mg |
| Dydrogesteron | 5 bis 50 mg |
| Medrogeston | 2 bis 30 mg |
| Ethynodiol, -diacetat | 0,4 bis 3 mg |
| Promegeston | 0,5 bis 10 mg |
| Nomegestrolacetat | 0,5 bis 10 mg |
| Trimegeston | 0,1 bis 10 mg |
| Etonogestrel | 0,1 bis 1 mg |
| Norelgestromin | 0,1 bis 2 mg |
| Norethynodrel | 0,3 bis 3 mg |
| Tibolon | 1 bis 10 mg |

Vorzugsweise weisen die erfindungsgemäßen Darreichungsformen, insbesondere die hormonhaltigen Tages-Einheiten, folgende Hormonkombinationen auf:
- 1.: 0,015 mg Ethinylestradiol +
0,06 mg Gestoden
- 2.: 0,02 mg Ethinylestradiol +
0,15 mg Desogestrel
- 3.: 0,02 mg Ethinylestradiol +
0,5 mg Norethisteron
- 4.: 0,02 mg Ethinylestradiol +
1 mg Chlormadiononacetat oder 2 mg oder 3 mg Chlormadinonacetat
- 5.: 0,02 mg Ethinylestradiol +
1 mg Norethisteron
- 6.: 0,03 mg Ethinylestradiol +
1 mg Norethisteron
- 7.: 0,02 mg Ethinylestradiol +
4 mg Chlormadinonacetat
- 8.: 0,02 mg Ethinylestradiol +
5 mg Chlormadinonacetat
- 9.: 0,02 mg Ethinylestradiol +
0,1 mg Levonorgestrel
- 10.: 0,02 mg Ethinylestradiol +
0,15 mg Desogestrel
- 11.: 0,02 mg Ethinylestradiol +
0,1 mg Levonorgestrel
- 12.: 0,03 mg Ethinylestradiol +
3 mg Drospirenon
- 13.: 0,02 mg Ethinylestradiol +
3 mg Drospirenon
- 14.: 0,03 mg Ethinylestradiol +
2 mg Chlormadinonacetat
- 15.: 0,035 mg Ethinylestradiol +
0,25 mg Norgestimat
- 16.: 0,03 mg Ethinylestradiol +
0,5 mg Norethisteron
- 17.: 0,03 mg Ethinylestradiol +
0,15 mg Desogestrel
- 18.: 0,03 mg Ethinylestradiol +
0,075 mg Gestoden
- 19.: 0,03 mg Ethinylestradiol +
0,15 mg Levonorgestrel
- 20.: 0,03 mg Ethinylestradiol +
0,15 mg Desogestrel
- 21.: 0,03 mg Ethinylestradiol +
0,15 mg Levonorgestrel
- 22.: 0,03 mg Ethinylestradiol +
0,125 mg Levonorgestrel
- 23.: 0,0375 mg Ethinylestradiol +
0, 75 mg Lynestrenol
- 24.: 0,03 mg Ethinylestradiol +
1 mg Norethisteron
- 25.: 0,03 mg Ethinylestradiol +
0,5 mg Norethisteron
- 26.: 0,03 mg Ethinylestradiol +
0,15 mg Levonorgestrel
- 27.: 0,04 mg Ethinylestradiol +
2 mg Lynestrenol
- 28.: 1. Phase = 7 Tage
0,050 mg Desogestrel + 0,035 mg Ethinylestradiol
2. Phase = 7 Tage
0,100 mg Desogestrel + 0,030 mg Ethinylestradiol
3. Phase = 7 Tage
0,150 mg Desogestrel + 0,030 mg Ethinylestradiol
- 29.: 1. Phase = 6 Tage
0,03 mg EE + 0.05 mg Levonorgestrel
2. Phase = 5 Tage
0,04 mg EE + 0,075 mg Levonorgestrel
3. Phase =10 Tage
0,03 mg EE + 0,125 mg Levonorgestrel
- 30.: 1. Phase = 7 Tage
0,035 mg EE + 0,180 mg Norgestimat
2. Phase = 7 Tage
0,035 mg EE + 0,215 mg Norgestimat
3. Phase = 7 Tage
0,035 mg EE + 0,250 mg Norgestimat
- 31.: 1. Phase = 7 Tage
0,035 mg EE + 0,5 mg Norethisteron
2. Phase = 9 Tage
0,035 mg EE + 1 mg Norethisteron
3. Phase = 5 Tage
0,035 mg EE + 0,5 mg Norethisteron
- 32.: 1. Phase = 7 Tage
0,035 mg EE + 0,5 mg Norethisteron
2. Phase = 7 Tage
0,035 mg EE + 0,75 mg Norethisteron
3. Phase = 7 Tage
0,035 mg EE + 1 mg Norethisteron
- 33.: 1. Phase = 6 Tage
0,03 mg EE + 0,05 mg Levonorgestrel
2. Phase = 6 Tage
0,04 mg EE + 0,075 mg Levonorgestrel
3. Phase = 9 Tage
0,03 mg EE + 0,125 mg Levonorgestrel
- 34.: 1. Phase = 6 Tage
0,03 mg EE + 0,05 mg Levonorgestrel
2. Phase = 5 Tage
0,05 mg EE + 0,05 mg Levonorgestrel
3. Phase = 10 Tage
0,04 mg EE + 0,125 mg Levonorgestrel
- 35.: 0,035 mg Ethinylestradiol +
2 mg Cyproteronacetat
- 36.: 0,05 mg Mestranol +
2 mg Chlormadinonacetat
- 37.: 1. Phase = 11 Tage
0,05 mg Ethinylestradiol +
1 mg Chlormadinonacetat
2. Phase = 11 Tage
0,05 mg Ethinylestradiol + 2 mg Chlormadinonacetat
- 38.: 0,08 mg Mestranol +
2 mg Chlormadinonacetat
- 39.: 0,03 mg Ethinylestradiol +
2 mg Dienogest
- 40.: 0,05 mg Ethinylestradiol +
0,5 mg Norgestrel
- 41.: 0,05 mg Ethinylestradiol +
0,125 mg Levonorgestrel
- 42.: 0,05 mg Ethinylestradiol +
0,25 mg Levonorgestrel
- 43.: 0,05 mg Ethinylestradiol +
1 mg Norethisteronacetat
- 44.: 1. Phase = 7 Tage
0,04 mg Ethinylestradiol +
0,025 mg Desogestrel
2. Phase = 15 Tage
0,03 mg Ethinylestradiol +
0,125 mg Desogestrel
- 45.: 1. Phase = 11 Tage
0,05 mg Ethinylestradiol +
0,05 mg Levonorgestrel
2. Phase = 10 Tage
0,05 mg Ethinylestradiol +
0,125 mg Levonorgestrel
- 46.: 1. Phase = 7 Tage
0,05 mg Ethinylestradiol

2. Phase = 15 Tage
0,05 mg Ethinylestradiol +
2,5 mg Lynestrenol
- 47.: 1. Phase = 7 Tage
0,05 mg Ethinylestradiol
2. Phase = 15 Tage
0,05 mg Ethinylestradiol +
0,125 mg Desogestrel
- 48.: 1. Phase = 6 Tage
0,05 mg Ethinylestradiol
2. Phase = 15 Tage
0,05 mg Ethinylestradiol +
1 mg Norethisteronacetat

Es wird empfohlen, die erfindungsgemäße Darreichungsform, sofern sie eine mehrphasige Hormonkombination vorsieht, nur für eine ununterbrochene Einnahme der hormonhaltigen Tages-Einheiten für die Dauer von 21 bis 25 Tagen, gefolgt von einer 7- bis 3-tägigen Einnahme von hormonfreien Tages-Einheiten vorzusehen.

Vorzugsweise weisen die hormonhaltigen Tages-Einheiten 5 bis 30 µg Ethinylestradiol und 0,5 bis 5 mg Chlormadinonacetat auf, wobei mindestens 21 Tages-Einheiten, vorzugsweise 21 bis 25 Tages-Einheiten und 3 bis 7 hormonfreie Tages-Einheiten, in der erfindungsgemäßen Darreichungsform vorliegen. Die erfindungsgemäße Darreichungsform kann aber auch hormonhaltige Tages-Einheiten für mehrere Jahre, vorzugsweise von 42 bis 365 Einheiten umfassen, die die genannte Hormonkombination in den angegebenen Mengenbereichen enthalten, wobei sich an die entsprechenden ununterbrochenen Einnahmezeiten 7 bis 3 hormonfreie Tages-Einheiten mit einer erfindungsgemäß angegebenen, erhöhten Menge Folsäure anschließen.

Wie bereits ausgeführt, können die hormonhaltigen Tages-Einheiten und die hormonfreien Tages-Einheiten der erfindungsgemäßen Darreichungsform als ein-(mono) oder mehrphasiges Kontrazeptivum aufgebaut sein. Bei einem mehrphasigen Kontrazeptivum kann eine Zweiphasen- oder eine Dreiphasen-Pille vorliegen, die sich aber nicht üblicherweise für eine über den natürlichen, weiblichen Zyklus hinausgehende Einnahmedauer eignet.

Die erfindungsgemäße Darreichungsform umfasst die Tages-Einheiten vorzugsweise in Form von Tabletten, die im Blister verpackt sind, die auch vorzugsweise eine Einnahme-Markierung aufweisen. Dies ist insbesondere von Vorteil, wenn die erfindungsgemäße Darreichungsform als ein Kontrazeptivum entsprechend dem weiblichen Menstruationszyklus vorgesehen ist.

Die erfindungsgemäße Darreichungsform kann auch ein Bestandteil eines Kits sein, wobei das erfindungsgemäße Kit mehrere der erfindungsgemäßen Darreichungsformen umfassen kann, insbesondere wenn eine Darreichungsform nur einen monatlichen Zyklus umfasst. Gegebenenfalls wird von dem Kit auch noch ein Kalender bzw. ein Kalenderbuch mit umfasst.

### Beispiele

### Beispiel 1:

### Zusammensetzung

| | a) | b) |
|---|---|---|
| | Pro Tablette | Pro Tablette |
| Ethinylestradiol | 0,020 mg | |
| Chlormadinonacetat | 2,000 mg | |
| Natriumfolat | 0,050 mg | 3,000 mg |
| Povidon K30 | 3,000 mg | 3,000 mg |
| Lactose | 31,930 mg | 31,000 mg |
| Maisstärke | 12,000 mg | 12,000 mg |
| Magnesiumstearat | 0,500 mg | 0,500 mg |
| hochdisperses Siliciumdioxid | 0,500 mg | 0,500 mg |

a) Ethinylestradiol (EE), Povidon K 30 (Polyvinylpyrrolidon) und das Natriumsalz der Folsäure wurden in 600 ml Ethanol gelöst. Chlormadinonacetat (Partikelgröße 90% < 50 µm), Lactose und Maisstärke wurden in einem Mischer/Granulierer (Diosna P25) 5 min gemischt und anschließend mit der ethanolischen EE/PVP Lösung durchfeuchtet und gemischt. Die feuchte Masse wurde durch ein 3 mm Sieb getrieben und in einem Vakuumtrockenschrank getrocknet. Das trockene Granulat wurde durch ein 0,6 mm Sieb desagglomeriert, mit Magnesiumstearat und hochdispersem Siliciumdioxid gemischt und auf einer Tablettenpresse mit 5 mm Stempeln zu Tabletten mit einem Gewicht von 50 mg gepresst.
b) Wie unter a) angegeben, wurden hormonfreie, folsäurehaltige Tabletten mit einem Gewicht von 50 mg hergestellt, wobei das Natriumsalz der Folsäure in 600 ml wässrigem Ethanol gelöst wurde.

Die Tabletten a) bzw. b) wurden mit einem Lack auf Basis Methylhydroxypropylcellulose überzogen (z. B. Opadry YS-1-2184 vom Hersteller Colorcon); Überzugsmasse 2 mg pro Tablette.

Zur Herstellung eines erfindungsgemäßen Kontrazeptivums wurden 21 hormonhaltige Tabletten und 7 hormonfreie Tabletten als 28 Tages-Einheiten zu einem Blister verpackt.

## Patentansprüche

1. Darreichungsform zur hormonalen Kontrazeption enthaltend eine bestimmte Anzahl von hormonhaltige Tages-Einheiten und eine bestimmte Anzahl von hormonfrelen Tages-Einheiten zur ununterbrochenen, täglichen, oralen Verabreichung an Frauen, **dadurch gekennzeichnet, dass** die hormonhaltigen Tages-Einheiten Folsäure in einer Tagesmenge bis höchstens 200 µg und die hormonfreien Tages-Einheiten jeweils Folsäure in einer Tagesmenge > 200 µg bis zu der bei Frauen maximal zulässigen Menge an Folsäure enthalten.

2. Darreichungsform nach Anspruch 1, **dadurch gekennzeichnet, dass** die hormonhaltigen Tages-Einheiten jeweils 5 bis 200 µg Folsäure enthalten.

3. Darreichungsform nach Anspruch 1, **dadurch gekennzeichnet, dass** die hormonhaltigen Tages-Einheiten keine Folsäure enthalten.

4. Darreichungsform nach Anspruch 1, **dadurch gekennzeichnet, dass** die hormonhaltigen Tages-Einheiten Folsäure bis zur minimal wirksamen Tagesmenge bei Frauen enthalten.

5. Darrelchungsform nach einem der Ansprüche 1 bis 4, **dadurch gekennzeichnet, dass** die hormonfreien Tages-Einheiten jeweils mehr als 200 µg bis zu 5 mg Folsäure enthalten.

6. Darreichungsform nach einem der Ansprüche 1 bis 5, **dadurch gekennzeichnet, dass** die hormonhaltigen Tages-Einheiten jeweils dieselbe Menge Folsäure und die hormonfreien Tages-Einheiten ebenfalls jeweils dieselbe Menge Folsäure enthalten.

7. Darreichungsform nach einem der Ansprüche 1 bis 6, **dadurch gekennzeichnet, dass** sie wenigstens 21 hormonhaltige Tages-Einheiten und 7 bis 3 hormonfreie Tages-Einheiten aufweist.

8. Darreichungsform nach Anspruch 7, **dadurch gekennzeichnet, dass** deren maximale Anzahl an hormonhaltigen Tages-Einheiten einer ununterbrochenen Verabreichung für mehrere Jahre, vorzugsweise für 2 Jahre, besonders bevorzugt für 1 Jahr, und deren Anzahl an hormonfreien Tages-Einheiten einer Verabreichung für 7 bis 3 Tage entspricht.

9. Darreichungsform nach Anspruch 7, **dadurch gekennzeichnet, dass** sie bis zu 730, vorzugsweise bis zu 365 hormonhaltige Tages-Einheiten und 7 bis 3 hormonfreie Tages-Einheiten aufweist.

10. Darreichungsform nach Anspruch 7, **dadurch gekennzeichnet, dass** sie 21 bis 25 hormonhaltige Tages-Einheiten und 7 bis 3 hormonfreie Tages-Einheiten aufweist.

11. Darreichungsform nach Anspruch 7, **dadurch gekennzeichnet, dass** sie 42 bis 52 hormonhaltige Tages-Einheiten und 7 bis 3 hormonfreie Tages-Einheiten aufweist.

12. Darreichungsform nach Anspruch 7, **dadurch gekennzeichnet, dass** sie 77 bis 193 hormonhaltige Tages-Einheiten und 7 bis 3 hormonfreie Tages-Einheiten aufweist.

13. Darreichungsform nach einem der Ansprüche 1 bis 12, **dadurch gekennzeichnet, dass** die hormonhaltigen Tages-Einheiten jeweils einen Gehalt an mindestens einer kontrazeptiv wirkenden Hormonkomponente, vorzugsweise einer Kombination von Hormonkomponenten, besonders bevorzugt eine Kombination aus einem Östrogen und einem Gestagen, aufweisen.

14. Darreichungsform nach einem der Ansprüche 1 bis 13, **dadurch gekennzeichnet, dass** die Anzahl der hormonhaltigen Tages-Einheiten einem monophasigen Kontrazeptivum entspricht.

15. Darreichungsform nach Anspruch 13, **dadurch gekennzeichnet, dass** eine hormonhaltige Tages-Einheit ein Östrogen aus der Gruppe umfassend Oestradiol, Estradiolvalerat, Ethinylestradiol, Mestranol und ein Gestagen aus der Gruppe umfassend Norethisteron, Norethisteronacetat, Norethisteronenantat, Norgestimat, Norgestrel, Levonorgestrel, Gestoden, Hydroxyprogesteroncaproat, Medroxyprogesteronacetat, Megestrolacetat, Chlormadinonacetat, Lynestrenol, Cyproteronacetat, Drospirenon, Dienogest, Desogestrel, Progesteron, Dydrogesteron, Medrogeston, Ethynodiol, Promegeston, Nomegestrolacetat und Trimegeston enthält.

16. Darreichungsform nach Anspruch 14, **dadurch gekennzeichnet**, das eine hormonhaltige Tages-Einheit eine Hormonkombination ausgewählt aus der Gruppe umfassend
1. 0,015 mg Ethinylestradiol +
0,06 mg Gestoden
2. 0,02 mg Ethinylestradiol +
0,15 mg Desogestrel
3. 0,02 mg Ethinylestradiol +
0,5 mg Norethisteron
4. 0,02 mg Ethinylestradiol +
1 mg Chlormadiononacetat
oder 2 mg oder 3 mg
Chlormadinonacetat
5. 0,02 mg Ethinylestradiol +
1 mg Norethisteron
6. 0,03 mg Ethinylestradiol +
1 mg Norethisteron
7. 0,02 mg Ethinylestradiol +
4 mg Chlormadinonacetat
8. 0,02 mg Ethinylestradiol +
5 mg Chlormadinonacetat
9. 0,02 mg Ethinylestradiol +
0,1 mg Levonorgestrel
10. 0,02 mg Ethinylestradiol +
11. 0,02 mg Ethinylestradiol +
0,1 mg Levonorgestrel
12. 0,03 mg Ethinylestradiol +
3 mg Drospirenon 13. 0.02 mg Ethinylestradiol +
3 mg Drospirenon
14. 0,03 mg Ethinylestradiol +
2 mg Chlormadinonacetat
15. 0,035 mg Ethinylestradiol +
0,25 mg Norgestimat
16. 0,03 mg Ethinylestradiol +
0,5 mg Norethisteron
17. 0,03 mg Ethinylestradiol +
0,15 mg Desogestrel
18. 0,03 mg Ethinylestradiol +
0,075 mg Gestoden
19. 0,03 mg Ethinylestradiol +
0,15 mg Levonorgestrel
20. 0,03 mg Ethinylestradiol +
0,15 mg Desogestrel
0,15 mg Desogestrel
22. 0,03 mg Ethinylestradiol +
0,125 mg Levonorgesirel
23. 0,0375 mg Ethinylestradiol +
0,75 mg Lynestrenol
24. 0,03 mg Ethinylestradiol +
1 mg Norethisteron
21. 0,03 mg Ethinylestradiol +
0,15 mg Levonorgestrel
25. 0,03 mg Ethinylestradiol +
0,5 mg Norethisteron
26. 0,03 mg Ethinylestradiol +
0,15 mg Levonorgestrel
oder
27. 0,04 mg Ethinylestradiol +
2 mg Lynestrenol
enthält

17. Ein Kit enthaltend mindestens eine Darreichungsform zur hormonalen Kontrazeption nach einem der Ansprüche 1 bis 16.

18. Ein Kit nach Anspruch 17, **dadurch gekennzeichnet, dass** es mehrere Darreichungsformen nach einem der Ansprüche 1 bis 16 enthält.
